# EUROPEAN PATENT APPLICATION

(11) **EP 2 745 830 A1**
(43) Date of publication of application: **25.06.2014**
(21) Application number: 12198227.6
(22) Date of filing: 19.12.2012
(51) Int. Cl.: A61K 8/25, A61Q 19/00, A61K 8/02, A61K 8/19

(54) **Method for reducing skin incomfort**

(71) Applicant: Solvay SA, 1120 Bruxelles (BE)
(72) Inventor: Patat, Olivier, 75017 Paris (FR); Marquez Sequeira Campos, Juan Antonio, 75018 Paris (FR); Palangie, Nicolas, 02300 Saint Aubin (FR)
(74) Representative: Vande Gucht, Anne

(57) **Abstract**

The invention relates to an aqueous composition for cosmetic or pharmaceutical use, in particular dermatogical use, to be applied on skin, comprising more than 20% by weight of alkali metal bicarbonate wherein the alkali metal bicarbonate is present in dissolved form and in particles form and the particles have a particle size distribution such that at least 50% by weight of the particles have a diameter of less than 100 µm for calming skin discomfort. The invention also relates to a method for improving the surface appearance of the skin and/or for calming the skin and in particular for reducing skin discomfort.

## Description

### Field of the invention

The invention relates to a method for the non-therapeutic cosmetic treatment of the skin, in particular to a method for improving the surface appearance of the skin and/or for calming the skin and in particular for reducing skin discomfort.

The invention relates also to a composition for cosmetic or pharmaceutical use, in particular dermatological use, to be applied on skin for calming skin discomfort and improving dryness and roughness, particularly suitable for psoriatic skin, ichthyotic skin, hyperkeratotic skin and/or dry skin.

### Prior art

Among its many physiological functions, the epidermis possesses the function of constituting a simultaneously biological, physical and chemical barrier against the invasion of the body by microorganisms. The physical barrier properties of the epidermis are especially linked to its structural organization. Many skin disorders may result from a dysfunction of the epidermal cellular structural organization or from a defect in the chemical barrier function of the epidermis, or from various attacks of environmental, biological or chemical origin.

Skin reactions may be manifested in the form of a discomfort felt in the skin. These may typically be redness, itching, stinging sensations or tautness. Skin disorders may be manifested as psoriasis, skin dryness, atopic dermatitis, ichthyosis or hyperkeratosis.

Psoriasis, for example, is manifested by red plaques due to the excessively rapid renewal of the epidermal cells, in only four to six days instead of about twenty days customarily, which causes localized inflammations. The epidermal cells accumulate at the surface of the skin and form a layer of white flakes known as scales. These are perfectly harmless, but they have the disadvantage of being unsightly and of causing discomfort through itching. Psoriasis plaques are often located on the elbows, knees, scalp and lower back, and may reach any other part of the body such as the face, hands, feet, skin folds and nails. Although this disease rarely endangers a person's life, it does on the other hand have a significant impact on a person's quality of life, given its unsightly appearance and its chronicity.

To date, conventional local treatments are available, such as dermocorticoids, tars, anthralin and retinoids, some of which have a large number of drawbacks and side effects.

US 2003/0228374 discloses a method and composition for treating seborrehic dermatitis of scalp, dandruff and psoriasis. The method includes topically applying to the skin a solution comprising 0,3 to 75% by to volume of one or more alkaline buffers such as bicarbonate, carbonate, di- or tri-basic phosphate of sodium or potassium. The document is silent on the presence of solid particles in the solution. Use of common pharma grade of such buffers at high concentration leads to abrasive irritating compositions.

Therefore there is a need to have novel or improved cosmetic treatment methods that can be used for relieving and/or treating these skin disorders.

The object of the present invention is to resolve at least one of the aforementioned drawbacks.

### Brief summary of the invention

Within this context, the inventors have shown, unexpectedly, that a method according to the present invention makes it possible to improve the appearance of the skin and to calm skin discomfort. It is moreover quick to use and uses a small amount of products.

Surprisingly and unexpectedly, the applicant has been able to demonstrate an improvement of the appearance of the skin, and a calming activity after topical application of the aqueous composition. Indeed, the tests that are presented in the examples of the present invention demonstrate the improvement of the appearance of the skin, and the calming activity. Due to this cosmetic and calming effect, the advantage of using the aqueous composition as a calmative is easily understood.

Moreover it has been observed that the effect of the efficacy of the composition on a skin area to be treated is non linear versus the time of contact of it with the skin area.

The composition may be intended for a cosmetic or pharmaceutical, particularly dermatological, application. Preferably the composition is intended for a non-therapeutic cosmetic application. The composition may be applied to the skin (to any cutaneous area of the body) and/or to the scalp.

The present invention therefore relates to a method for the non-therapeutic cosmetic treatment of the skin according to Claim 1.

The present invention also relates to an aqueous composition for cosmetic or pharmaceutical use, in particular dermatological use, according to Claim 6 to be applied on skin.

The present invention also relates to a non-therapeutic use of an aqueous cosmetic composition according the invention for improving the surface appearance of the skin.

The present invention also relates to a non-therapeutic use of an aqueous cosmetic composition according the invention as a calmative.

The present invention therefore proposes the use of a composition according the invention which thus makes it possible to reduce the skin discomfort felt by a subject (generally a human being).

The method and uses according to the invention make it possible to calm and/or to relieve and/or to combat, in a mild manner, skin discomfort such as itching, tautness, pruritus, tingling, stinging, irritation, redness, the sensation of heat, and the symptoms of sensitive and/or irritable and/or reactive and/or intolerant skin and/or scalps.

Other aspects, distinctive features and advantages of the present invention will become apparent on reading the description which follows and the examples given purely by way of illustration and which could in no way limit the scope of the invention.

### Brief description of the figures

Figure 1 represents a graph showing the change in the TSS subscores after 1 application of the gel prepared in Example 1b.
Figure 2 represents a graph showing the change in the TSS total score after 1 application of the gel prepared in Example 1b.
Figure 3 represents a graph showing the change in the other clinical signs after 1 application of the gel prepared in Example 1b.
Figure 4 represents a graph showing the change in the TSS subscores after 5 applications of the gel prepared in Example 1b.
Figure 5 represents a graph showing the change in the TSS total score after 5 applications of the gel prepared in Example 1b.
Figure 6 represents a graph showing the change in the other clinical signs after 5 applications of the gel prepared in Example 1b.
Figure 7 represents two photos of the treated area before (D0) and after (D9) 5 applications of the gel prepared in Example 1b.

### Detailed description of the invention

The treatment method and use according to the invention have in particular the features of a cosmetic method and use since they make it possible to improve the aesthetics (appearance) and/or the comfort of the skin, and to improve the comfort of an individual, in particular the skin comfort of an individual. The treatment methods and uses of the invention are particularly intended for humans.

The expression "cosmetic composition" denotes here a composition intended to provide a cosmetic effect within the context of a topical application.

The present invention relates to a method for the non-therapeutic cosmetic treatment of the skin, comprising the application to the skin of an aqueous composition comprising more than 20% by weight of alkali metal bicarbonate, advantageously at least 25%, preferably at least 29%, more preferably at least 30% by weight of alkali metal bicarbonate relative to the total weight of the composition, wherein the alkali metal bicarbonate is present in dissolved form and in particles form and the particles have a particle size distribution such that at least 50% by weight of the particles have a diameter of less than 100 µm. Advantageously the particles have a particle size distribution such that at least 75% by weight of the particles have a diameter of less than 100 µm. Preferably the particles have a particle size distribution such that at least 90% by weight of the particles have a diameter of less than 100 µm.

The alkali metal bicarbonate may, for example, be bicarbonate in the strict sense such as potassium bicarbonate or sodium bicarbonate. However, it also covers compound salts such as alkali metal sesquicarbonates which comprise bicarbonate. Sodium or potassium bicarbonates are particularly suitable. Preferably, bicarbonates in the strict sense are used. Potassium bicarbonate or sodium bicarbonate, more particularly sodium bicarbonate, are preferred. The percentages of weight of the compositions are relative to the total weight of the composition. A composition comprising more than 85% by weight of alkali metal bicarbonate has, in general, a solid appearance that is not very easy to spread on the skin. Thus, it is advantageous for the aqueous composition to contain at most 80%, preferably at most 65%, and more preferably at most 55% by weight of alkali metal bicarbonate. Compositions comprising at most 55%, preferably at most 40%, and more preferably at most 35% by weight of alkali metal bicarbonate enable easier manufacture of aqueous composition comprising compounds such as silica, gelling agent, moisturizing agent, emollient, and thickeners. It also eases the homogenization of compositions when in gel or cream form.

According to the invention, the alkali metal bicarbonate present in particles form in the aqueous composition comprises particles having a particle size distribution such that at least 50%, advantageously 75%, preferably 90% by weight of the particles have a diameter of less than 70 µm. The average diameter is advantageously less than 50 µm, preferably 40 µm The diameters are measured according to the standard ASTM C-690-1992. The remainder of the bicarbonate, which is not in the form of particles, is in dissolved form. Such particles size distributions have shown an improved effect for calming skin discomfort. As they decrease skin irritations in particular on: psoriatic skin, ichthyotic skin, hyperkeratotic skin and/or dry skin. The aqueous composition may easily be obtained by mixing, with water, a bicarbonate powder having a suitable particle size, considering the fact that at the moment of mixing, the fine particles preferably go into solution and most of coarser particles remain.

The aqueous composition according to the method of the invention, comprises generally at least 20%, advantageously at least 30%, more advantageously at least 35% by weight of water relative to the total weight of the composition. It comprises generally at most 80%, preferably at most 70% by weight of water relative to the total weight of the aqueous composition. Aqueous compositions in gel form comprise advantageously 50% to 65%, preferably 55% to 65% of water by weight. Aqueous compositions in emulsion (cream) form comprise advantageously 20% to 45%, preferably 35% to 40% of water by weight.

In one preferred embodiment of the method according to the invention, the composition also comprises at least 1%, advantageously at least 3%, preferably at least 5%, more preferably at least 7%, most preferably at least 10% by weight of silica relative to the total weight of the composition. Too large an amount of silica in the composition is undesirable since the cost of silica is high, it makes the composition dustier once dried and generates more insoluble materials during removal, whereas the alkali metal bicarbonate dissolves easily in water and is transported in the drainage systems of dwellings or hospitals without leaving any traces. It is recommended that the composition does not comprise more than 20%, preferably at most 15% by weight of silica relative to the total weight of the composition. According to an advantageous embodiment of the one preferred embodiment, the aqueous composition also comprises from 1 to 20% by weight of silica. According an even more advantageous embodiment of the one preferred embodiment of the method or of the composition, the composition comprises, or consists essentially of, or consists of: 25 to 40% of alkali metal bicarbonate, 10 to 20% by weight of silica, and water. It is recommended that the silica is in amorphous (and not crystalline) form for the tolerance thereof by the human body.

It is preferred for the silica to be in the form of very fine particles, having a high specific surface area. According to the preferred mode of the present invention, the specific surface area of the silica is in general at least 5 m²/g, advantageously at least 50 m²/g, preferably at least 100 m²/g and most preferably at least 200 m²/g, measured according to the standard ISO 5794-1, annex D. In one advantageous embodiment of the invention, the silica is in the form of particles having an average diameter of less than 10 µm. The average diameter is measured according to the standard ASTM C-690-1992.

The aqueous composition may be in any galenic form normally used for a topical application, especially in the form of a gel, a cream or an ointment.

In one variant of the method according to the invention, the composition is in the form of a gel. Gels are compositions comprising at least two components, generally a solid dispersed in colloidal form in a liquid phase. The dispersed particles form space lattices, stabilized by means of van der Waals forces. In hydrogels, the liquid phase is water. In this variant, it is advantageous for the composition to contain silica. When the composition contains silica it is particularly easy to produce a gel by simple addition of water to the bicarbonate-silica mixture.

The invention therefore also relates to an aqueous composition in the form of a gel comprising from 20% to 55%, preferably from 25% to 40%, preferably from 25% to 35% by weight of alkali metal bicarbonate particles relative to the total weight of the composition, from 5% to 20%, preferably from 10% to 20% by weight of silica relative to the total weight of the composition and from 30% to 60%, preferably 40% to 60%, preferably 50% to 60% by weight of water relative to the total weight of the composition. Preferably, the composition in the form of a gel comprises from 29% to 35% by weight of alkali metal bicarbonate particles relative to the total weight of the composition, from 11% to 15% by weight of silica relative to the total weight of the composition and from 53% to 57% by weight of water relative to the total weight of the composition.

The aqueous composition according the invention may also comprise gel-forming polymers such as polysaccharides, pectin, xanthan, etc... Particularly advantageous aqueous composition comprising gel-forming polymers is described by Claim 10. Such composition has proven to be a particularly stable composition, with low or no decantation of the alkaline metal bicarbonate particles in the aqueous composition, and having particularly effective properties for calming skin discomforts according present invention.

Preferably, the aqueous composition according to Claim 10 comprises, or consists essentially of, or consists of:
- 25 to 40% of alkali metal bicarbonate,
- 1 to 3% of gelling agent such as polysaccharides, glycoproteins, xanthan gum, acacia gum, xylitylglucoside, arabinose, ribose,
- optionally 1 to 15% of silica,
- 1 to 6% of moisturizing agent such as glycerine, anhydroxylitol, xylitol,
- QSP water to 100% by weight.

More preferably the aqueous composition according to Claim 10 comprises, or consists essentially of, or consists of:
- 25 to 35% of alkali metal bicarbonate,
- 1 to 3% of gelling agent such as polysaccharides, glycoproteins, xanthan gum, acacia gum, xylitylglucoside, arabinose, ribose,
- optionally 1 to 15% of silica,
- 1 to 6% of moisturizing agent such as glycerine, anhydroxylitol, xylitol,
- QSP water to 100% by weight.

Advantageously such aqueous composition is in a gel form and comprises less than 2% of oil, preferably is essentially free of oil, and/or comprises less than 5% of organic acid or organic acid alkali metal salt, preferably is essentially free of organic acid other than glycoproteins. Indeed organic acids have a peeling effect that irritates skin and therefore may decrease the calming activity for treating skin discomfort.

The aqueous composition may also comprise the adjuvants that are customary in the fields considered, such as film-forming agents, gelling agents, thickeners, surfactants and mixtures thereof. As is known, the composition may also contain excipients such as preservatives, antioxidants, solvents, fragrances, fillers and UV screening agents. The amounts of these various adjuvants and/or excipients are those conventionally used in the fields considered, for example from 0.01 % to 20% by weight relative to the total weight of the composition. These excipients are preferably selected from the group consisting of amino acids and derivatives thereof, polyglycerols, esters, polymers and derivatives of cellulose, lanoline derivatives, phospholipids, lactoferrins, lactoperoxidases, sucrose-based stabilizers, vitamin E and derivatives thereof, natural and synthetic waxes, plant oils, triglycerides, unsaponifiable materials, phytosterols, plant esters, silicones and derivatives thereof, protein hydrolysates, jojoba oil and derivatives thereof, fat-soluble/water-soluble esters, betaines, aminoxides, plant extracts, saccharose esters, titanium dioxides, glycines and parabens, and more preferably from the group consisting of butylene glycol, steareth-2, steareth-21, glycol-15 stearyl ether, cetearyl alcohol, phenoxyethanol, methylparaben, ethylparaben, propylparaben, butylparaben, butylene glycol, natural tocopherols, glycerin, sodium dihydroxycetyl, isopropyl hydroxycetyl ether, glycol stearate, triisononanoin, octyl cocoate, polyacrylamide, isoparaffin, laureth-7, a carbomer, propylene glycol, glycerol, bisabolol, a dimethicone, sodium hydroxide, PEG-30 dipolyhydroxystearate, capric/caprylic triglycerides, cetearyl octanoate, dibutyl adipate, grape seed oil, jojoba oil, magnesium sulphate, EDTA, a cyclomethicone, xanthan gum, sodium lauryl sulphate, mineral waxes and oils, isostearyl isostearate, propylene glycol dipelargonate, propylene glycol isostearate, PEG-8 Beeswax, hydrogenated palm kernel oil glycerides, hydrogenated palm oil glycerides, lanolin oil, sesame oil, cetyl lactate, lanolin alcohol, castor oil, titanium dioxide, lactose, saccharose, low-density polyethylene and an isotonic saline solution.

In one preferred embodiment of the method according to the invention, the aqueous composition comprises at least 20% by weight of alkali metal bicarbonate and also comprises at least one other film-forming component different from the alkali metal bicarbonate.

Mention will for example be made, as film-forming components, of polyethylene glycols, polysaccharides, derivatives of gums (such as guar, xanthan or carob gums), derivatives of polyglycerides and the mixtures of several of these compounds. Advantageously, said film-forming component is a polysaccharide selected from the group consisting of maltodextrins, dextrins, sorbitol, mannitol and xylitol; or is a polyethylene glycol having a weight-average molecular weight M_{w} ranging from 1000 g/mol to 6000 g/mol; or is a derivative of polyglyceride selected from the group consisting of lauroyl macrogolglycerides or stearoyl macrogolglycerides. The amount of film-forming components may vary for example from 0.01 % to 20% by weight relative to the total weight of the composition, for example from 0.05% to 15% by weight, for example from 0.1 % to 10% by weight.

The composition in the form of a gel or of a cream may be obtained, for example, by simple mixing of the constituents. Stable aqueous compositions in gel or cream form comprising solid particles of alkali metal bicarbonate are difficult to achieve when comprising oils and alkali metal bicarbonate particles. In particular, stable aqueous compositions in cream form are difficult to achieve when comprising oils and alkali metal bicarbonate particles. More particularly when natural products are used. Indeed no non-ionic surfactants have been found to stabilize efficiently such emulsions. After a centrifugation at 4000 rpm during 20 minutes, all the compositions tested with non-ionic surfactants present phases separation. On the contrary, the use of anionic surfactant in the range from 0,1 to 2% enables to stabilize emulsions made of aqueous compositions according the invention.

Particular advantageous embodiment of such creams or emulsions is described in Claim 12. Oil may be chosen advantageously from plant oils or natural oils such as: almond oil, jojoba oil, grape seed oil, sesame oil, apricot kernel oil, sunflower oil, coconut oil, avocado oil, macadamia oil, rosehip seed oil, castor oil, and mixtures thereof. Almond oil is preferred. Such compositions have proven to be particularly stable emulsion compositions when tested on a centrifuge, with reduced or no phase separation in presence of alkali metal bicarbonate, and having particularly effective properties for calming skin discomforts.

Preferably, the aqueous composition according to Claim 12 comprises, or consists essentially of, or consists of:
- 25 to 40% of alkali metal bicarbonate,
- 2 to 20% of oil such as almond oil,
- 3 to 10% of emulsifying agent such as Candilla/jojoba/rice bran polyglyceryl-3 esters and glyceryl stearate and cetearyl alcohol and sodium stearoyl lactylate,
- 0.1 to 2% of an anionic surfactant such as sodium lauroyl glutamate,
- optionally 1 to 15% of silica,
- 1 to 6% of moisturizing agent such as glycerine, anhydroxylitol, xylitol,
- 1 to 3% of gelling agent such as polysaccharides, glycoproteins, xanthan gum, acacia gum, xylitylglucoside, arabinose, ribose,
- QSP water to 100% by weight.

More preferably, the aqueous composition according to Claim 12 comprises, or consists essentially of, or consists of:
- 25 to 35% of alkali metal bicarbonate,
- 2 to 20% of oil such as almond oil,
- 3 to 10% of emulsifying agent such as Candilla/jojoba/rice bran polyglyceryl-3 esters and glyceryl stearate and cetearyl alcohol and sodium stearoyl lactylate,
- 0.1 to 2% of an anionic surfactant such as sodium lauroyl glutamate,
- optionally 1 to 15% of silica,
- 1 to 6% of moisturizing agent such as glycerine, anhydroxylitol, xylitol,
- 1 to 3% of gelling agent such as polysaccharides, glycoproteins, xanthan gum, acacia gum, xylitylglucoside, arabinose, ribose,
- QSP water to 100% by weight.

The present invention relates to a method according to Claim 1 for the non-therapeutic cosmetic treatment of the skin, comprising the application to the skin of the aqueous composition. Preferably, the method comprises the application to psoriatic skin, ichthyotic skin, hyperkeratotic skin and/or dry skin, preferably to psoriatic skin.

The present invention also relates to a cosmetic non-therapeutic use of an aqueous composition according to Claim 6, for calming skin discomfort and/or for improving the surface appearance of the skin. The present invention also relates to an aqueous composition for cosmetic or pharmaceutical use, in particular dermatological use, according to Claim 6. Advantageously the particles have a particle size distribution such that at least 75% by weight of the particles have a diameter of less than 100 µm Preferably the particles have a particle size distribution such that at least 90% by weight of the particles have a diameter of less than 100 µm.

Unless otherwise indicated, within the context of the invention, the term "skin" is understood to mean any cutaneous surface of the body including the skin, extended to the scalp, and with the exclusion of the mucous membranes.

In one particular embodiment, the method, the uses and the aqueous composition according to the invention are particularly useful for improving the surface appearance of the skin, with the exclusion of the mucous membranes. In one preferred embodiment, the method, the uses, and the composition according to the invention are particularly useful for improving the surface appearance of psoriatic skin, ichthyotic skin, hyperkeratotic skin and/or dry skin.

In one particular embodiment, the method, the uses, and the composition according to the invention are particularly useful for calming skin discomfort. In one preferred embodiment, the method and the uses according to the invention are particularly useful for calming skin discomfort in the case of psoriatic skin, ichthyotic skin, hyperkeratotic skin and/or dry skin.

Within the meaning of the present invention, the expression "skin discomfort" is understood to mean itching, tautness, pruritus, tingling, stinging, irritation, redness, the sensation of heat, and the symptoms of sensitive and/or irritable and/or reactive and/or intolerant skin and/or scalps. The sensitive and/or irritable and/or reactive and/or intolerant skin and/or scalps may be characterized by symptoms such as for example subjective signs which may be essentially dysesthetic sensations, that is to say sensations felt in a cutaneous area such as itching, tautness, pruritus, tingling, stinging, irritation, etc.

In one preferred embodiment, the method and the composition according the invention are particularly advantageous for calming skin discomfort selected from itching, pruritus, tingling, tautness, and/or stinging. Preferably, the aqueous composition is applied to psoriatic skin, ichthyotic skin, hyperkeratotic skin and/or dry skin, more preferably psoriatic skin.

In the method for the cosmetic treatment of the aforementioned skin discomforts, in particular the symptoms of the skin and/or scalp, the cosmetic composition should be applied to the areas to be treated of an individual suffering from at least one of said skin discomforts and/or symptoms and is optionally left in contact for several minutes to several hours and is optionally rinsed; this being for uses that may be repeated or renewed over treatment periods ranging from a few days to several months or even several years.

In an advantageous embodiment the aqueous composition according the invention for treating psoriatic skin, ichthyotic skin, hyperkeratotic skin and/or dry skin, is applied on a skin area to be treated and is left in contact with the skin area at least 10 minutes, more advantageously at least 15 minutes, even more advantageously at least 30 minutes and is optionally rinsed. The inventors have observed that the effect of the efficacy of the composition on a skin area to be treated is non linear versus the time of contact of the composition with the skin area. A too short contact of one to five minutes show low efficacy. Contact time of at least 10, or better at least 15 or even better at least 30 minutes improves greatly the efficacy both for calming skin discomfort and for improving the surface appearance of the skin. Generally a contact time much larger than 30 minutes does not bring by much more efficacy. Preferably the aqueous composition according to Claim 13 is to be applied on a skin area to be treated and be left in contact with the skin area at least 10 minutes, advantageously at least 15 minutes, more advantageously at least 30 minutes and is optionally rinsed, and is optionally applied at least 5 times on a period of 9 days. Thus, the present invention also relates to a method for the cosmetic treatment of the skin, for calming at least one of the aforementioned skin discomforts, characterized by the fact that it comprises the application to the skin of an aqueous composition comprising at least 20% by weight of alkali metal bicarbonate, leaving the aqueous composition in contact with the skin, and optionally rinsing it off. The aqueous composition may advantageously be used as a calmative in the treatment of psoriasis, hyperkeratosis, of the parakeratosis, ichthyoses and dry skin.

The invention also relates to a cosmetic treatment method intended to improve the aesthetic appearance of the skin in the case of psoriasis, dry skin, hyperkeratosis, parakeratosis and/or ichthyosis, characterized by the fact that a cosmetic composition as described previously is applied to the skin. Advantageously, the method comprises the application to psoriatic skin, ichthyotic skin, hyperkeratotic skin and/or dry skin of the aqueous composition and leaving the latter in contact with the skin, and optionally rinsing it off. In one particular embodiment of the above cosmetic treatment method, the latter is intended to improve the aesthetic appearance of the skin with the exception of the scalp and/or with the exception of preventing and/or minimizing dandruff conditions.

The method and uses according to the invention are particularly useful for preventing and/or minimizing dandruff conditions. Thus, in another particular embodiment, the invention also relates to a method for the cosmetic treatment of the scalp characterized by the fact that it comprises the application to the scalp of an aqueous composition according the invention, in particular with a gel according to Claims 9 to 11. Preferably, the treatment is an antidandruff treatment. According to this particular embodiment, the composition may be formulated in the form of an antidandruff shampoo, medicated lotion, cream or styling gel.

The invention also relates to an aqueous composition according to Claims 6 to 14, for use as a calmative, preferably as a calmative for psoriatic skin, ichthyotic skin, hyperkeratotic skin and/or dry skin. The invention also relates to an aqueous composition for use for calming skin discomfort, for example for calming skin discomfort selected from itching, tautness, pruritus, tingling, stinging, irritation, redness, the sensation of heat, and the symptoms of sensitive and/or irritable and/or reactive and/or intolerant skin and/or scalps. The invention also relates to an aqueous composition according to Claims 6 to 14 for use for calming psoriatic skin, ichthyotic skin, hyperkeratotic skin and/or dry skin. Particularly in subjects or individuals suffering from psoriasis, hyperkeratosis, parakeratosis, ichthyosis and/or having dry skin. The aqueous composition may advantageously be used on psoriatic, hyperkeratotic, parakeratotic and/or ichthyotic lesions and/or on dry skin. The invention also relates to the use of an aqueous composition according to Claims 6 to 14 for the preparation of a calmative for calming skin discomfort in the case of psoriasis, dry skin, hyperkeratosis, parakeratosis and/or ichthyosis.

The following examples are given by way of non-limiting illustration of the present invention, and variants thereof that are readily accessible to a person skilled in the art are possible.

### Examples

### Example 1: Formulation of a topical gel based on sodium bicarbonate.

Example 1.a (non conform): A gel was prepared by mixing 31.5% of Bicar® Codex 0/50 pharmaceutical-grade sodium bicarbonate manufactured by Solvay, consisting of sodium bicarbonate particles having a particle size distribution such that at least 50% by weight of the particles have a diameter of more than 100 µm, and 13.5% by weight of Sylysia 290N amorphous silica manufactured by Silysiamont, and 55% of demineralized water.

Example 1.b (conform): A gel was prepared by mixing 31.5% of Bicar® Codex 0/13 pharmaceutical-grade sodium bicarbonate manufactured by Solvay, consisting of sodium bicarbonate particles having a particle size distribution such that at least 50% by weight of the particles have a diameter of less than 100 µm, and 13.5% by weight of Sylysia 290N amorphous silica manufactured by Silysiamont, and 55% of demineralized water.

The topical gels were produced from a powder, composed of 70% of sodium bicarbonate and 30% of synthetic amorphous precipitated silica, and of demineralized water.

To produce the topical gels, the alkali metal bicarbonate and the synthetic amorphous silica were premixed in order to form a mixture composed of 70% by weight of sodium bicarbonate and 30% by weight of silica in a Lödige stainless steel laboratory mixer with lifter blades in order to produce 900 g of mixture of the two powders.

1100 g of demineralized water were introduced into a glass reactor with stirring. Next, half of the total powder charge (i.e. 450 g) was introduced, as a fine rain, with stirring of around 300 rpm.

Next, the second half of the powder charge was introduced, as a fine rain, with a gradual increase of rotational speed of the stirring member. The rotational speed of the stirring member is thus brought, at the end of the introduction, to 1250 rpm. The total duration of the introduction operation was around 30 min. The mixture was then left stirring at 800 rpm for 1 hour.

**The** mixture was left at rest without stirring for a period of 1.5 hours.

Next the gels were put back under stirring for 20 min, then stirred for 3 min using a T 25 basic Ultra-Turrax disperser, equipped with its S25N25G stirring member, at a rotational speed of 9500 rpm.

Lastly, the gels were left to rest for around 8 hours before packaging. The gel 1.b exhibits an excellent stability over a time of several weeks.

### Example 2: Application of a topical gel based on sodium bicarbonate.

Example 2.a: The gels prepared in Example 1.a and 1.b were tested by a volunteer (1 man) affected by psoriasis as a pre-screening test. The evaluation of the tolerance of the gels was carried out on two separate psoriatic skin areas of the volunteer after 1 and 2 applications of 10 minutes each, before rincing, and the second application was realized 8 hours after the first application. The tolerance was judged to be quite good after 1 and 2 applications for gel 1.b (conform) on the treated area, and the tolerance was judged to be bad for gel 1.a (non conform) qualified as "irritating" on the treated area.

Example 2.b: The gel prepared in Example 1.b was tested by a group of volunteers over a total period of 10 days with a panel of 25 volunteers (16 men and 9 women) aged from 26 to 74 years old suffering from psoriasis for at least 6 months, without any oral treatment or with a stable treatment for at least 1 month and having visible cutaneous manifestations, in particular scales. The average age of the population studied was 54 years old +/- 17 years (min = 26 years old: max = 74 years old).

3 areas of study were determined at D0: a healthy "test area" not affected by psoriasis was determined, to which the product was applied under normal usage conditions. A "treated area" affected by psoriasis was selected, to which the gel of Example 1.b was applied. A "control area" affected by psoriasis, adjacent to the treated area, to which no product was applied, served as control area.

The application was carried out as a thick layer on a psoriatic area and on the test area for 30 minutes. After 30 minutes, the two areas were rinsed, then examined and photographed, and the intensity of the changes according to the TSS scale was measured.

The evaluation of the product was carried out at the start of the test (D30 minutes) and after 9 days (D9) of use of the gel. The evaluation of the effectiveness of the gel was carried out before, just after 1 application and after 5 applications across the following parameters: the TSS score (Total Severity Sign score), the observed effectiveness, and the appearance and size of the lesions analysed in photos.

The TSS score made it possible to evaluate 3 signs of psoriatic plaques: erythema, thickness and desquamation. The importance of the various signs observed over the various areas was rated from 0 to 4 according to the following scale:
- 0 = no sign,
- 1 = slight signs,
- 2 = moderate signs,
- 3 = severe signs,
- 4 = very severe signs.

The total score is the addition of the scores for erythema, thickness and desquamation, i.e. a total score which may be between 0 and 12. Upon inclusion, the desquamation score must be greater than or equal to 2 and the TSS total score must be greater than or equal to 4 on the "treated area" selected.

The symptomatology data were processed by the non-parametric Wilcoxon ranks test with, for null hypothesis H0: (A) = (B), and for the alternative hypothesis H1 : (A) ≠ (B), with (A): clinical symptomatology at D0 and (B): clinical symptomatology at D30 min (then D9). If the significance level obtained (p) was < 0.05, the null hypothesis H0 was rejected. Then an alternative hypothesis H1 of a significant difference between the clinical symptomatology observed at D30 min (then D9) and that at D0 was accepted. A comparison between the control area and the treated area was also made according to the same procedure each time. The software used for the statistical processing was the SPSS version 12.0 software. In the graphs, a level between 0.05 and 0.01 is marked by 1 star, between 0.01 and 0.001 by 2 stars and less than 0.001 by 3 stars. In the case of a significance of the test, the significance level is indicated in bold and underlined in the tables and in the case of a significance level between 0.10 and 0.05, it is in italics indicating a significant tendency.

Photographs of the treated area and of the control area were acquired at D0 and D9 using a Fotofinder Mediscope imaging platform. A distance bar made it possible to take the photos at the same distance and a "ghost" overlay of the previous photo allowed optimal reproducibility. The software used for the analyses of the photos was the HISTOLAB software.

At D0 and D9, the skin condition of the 3 areas was examined: before use, 30 minutes after application of the product and after 5 uses of the product. The clinical signs observed on the treated area, the control area and the test area for evaluating the tolerance were the following: erythema, dryness, roughness, desquamation, hyperkeratosis and pruritus, and also the reduction of the TSS score, the improvement of pruritus, the reduction of plaque size, and the improvement of plaque appearance.

For 100% of the volunteers, the product was well tolerated. The tolerance was judged to be good after 1 and 5 applications of the product on the control area and quite good on the treated area.

The effectiveness of the product after 1 application was verified on the main criterion of the total TSS score mainly by a significant improvement of the thickness and of the desquamation from the first application and by an improvement in significant tendency of the erythema. After one application, a significant reduction of the thickness (p = 0.003) and of the desquamation (p < 0.001) were observed and these reductions were significantly different from the change observed on the control area. The erythema had reduced with a significant tendency (p = 0.083) relative to D0 and relative to the control area. The total TSS score was significantly reduced on the treated area after one application relative to D0 (p < 0.001). Furthermore, the observed reduction of the TSS score on the treated area was significant relative to the change observed on the control area (p inter group < 0.001).

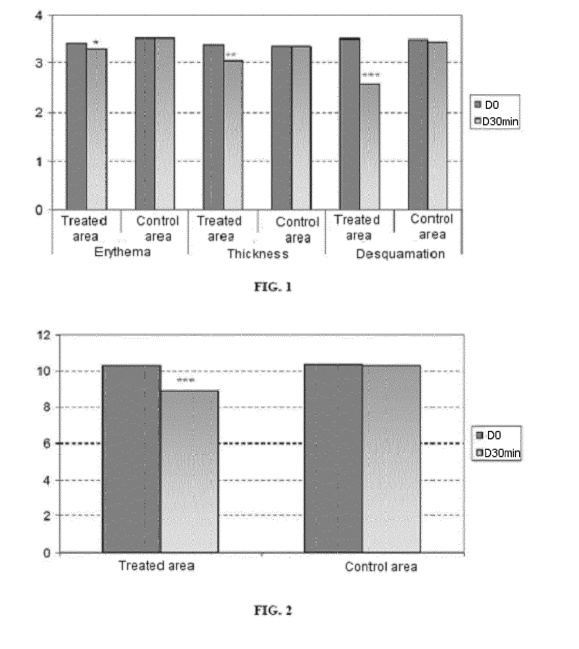

Table 1 indicates the average observed for each sign at D0 and D30 minutes on the treated area and the control area, the average difference observed between D0 and D30 minutes, and also the degree of significance of the Wilcoxon test of comparison of the averages on data coupled before and after 1 application (intra group significance) and the degree of comparison of the change between the treated area and the control area (inter group significance). Figures 1 and 2 represent these changes.

**Table 1**

| | Area | Av. D0 | Av. D30min | Delta/D0 | Intra sign./D0 | Inter sign./D0 |
|---|---|---|---|---|---|---|
| Erythema | Treated | 3.42 | 3.31 | -0.11 | *0.083* | *0.083* |
| | Control | 3.52 | 3.52 | 0 | 1 | |
| Thickness | Treated | 3.38 | 3.04 | -0.34 | **0.003** | **0.008** |
| | Control | 3.35 | 3.35 | 0 | 1 | |
| Desquamation | Treated | 3.50 | 2.58 | -0.92 | **<0.001** | **<0.001** |
| | Control | 3.48 | 3.44 | -0.04 | 0.317 | |
| TSS score | Treated | 10.31 | 8.92 | -1.39 | **<0.001** | **<0.001** |
| | Control | 10.35 | 10.30 | -0.05 | 0.317 | |

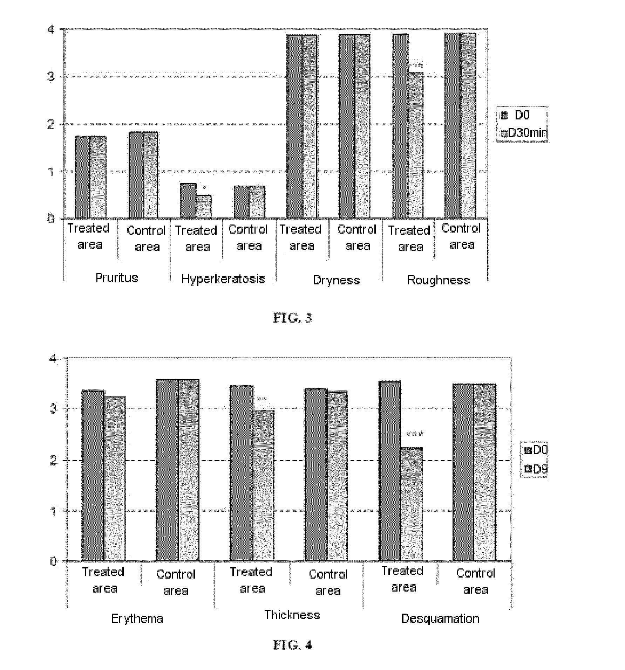

Table 2 indicates the average results of the other clinical signs at D0 and D30 minutes. Figure 3 represents the change in these other clinical signs at D0 and D30 minutes.

**Table 2**

| | Area | Av. D0 | Av. D30min | Delta/D0 | Intra sign./D0 | Inter sign./D0 |
|---|---|---|---|---|---|---|
| Pruritus | Treated | 1.73 | 1.73 | 0 | 1 | 1 |
| | Control | 1.83 | 1.83 | 0 | 1 | |
| Hyperkeratosis | Treated | 0.73 | 0.50 | -0.23 | **0.034** | *0.059* |
| | Control | 0.70 | 0.70 | 0 | 1 | |
| Dryness | Treated | 3.85 | 3.85 | 0 | 1 | 1 |
| | Control | 3.87 | 3.87 | 0 | 1 | |
| Roughness | Treated | 3.89 | 3.08 | -0.81 | **<0.001** | **<0.001** |
| | Control | 3.91 | 3.91 | 0 | 1 | |

On the treated area, the hyperkeratosis and the roughness decreased significantly after one application (p = 0.034 and p < 0.001 respectively). Compared to the change observed on the control area, these reductions were of significant tendency for the clinical sign of hyperkeratosis (p = 0.059) and significant for the roughness (p < 0.001). The total TSS score and the visual appearance of the plaques were improved in 92% of cases.

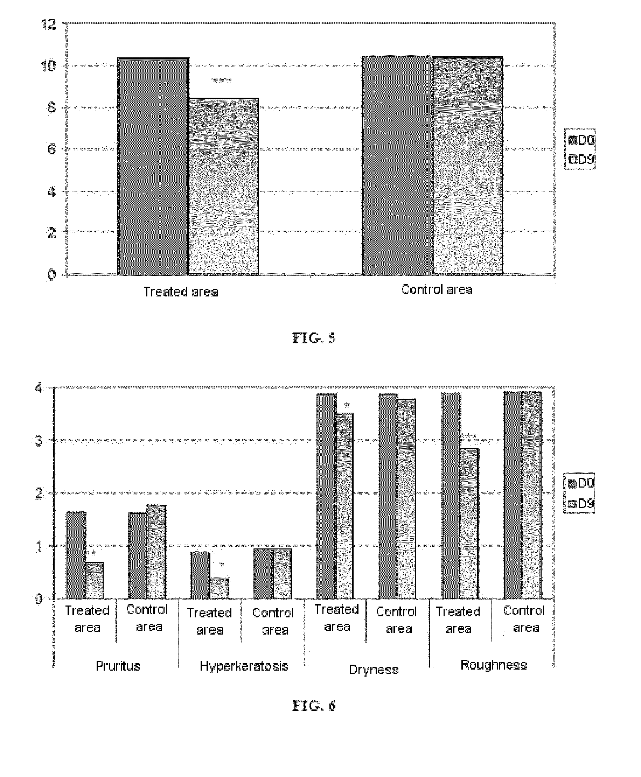

The effectiveness of the product after 5 applications was verified on the main criterion of the total TSS score mainly by a significant improvement of the thickness and of the desquamation. At D9, i.e. after 5 applications of the gel, the erythema decreased, but in a statistically insignificant manner on the treated area (p = 0.257) whereas the thickness and the desquamation decreased significantly (p = 0.002 and p < 0.001) as did the total TSS score (p < 0.001). Table 3 indicates the average observed for each sign at D0 and D9 on the treated area and the control area. Figures 4 and 5 represent these changes.

**Table 3**

| | Area | Av. D0 | Av. D9 | Delta/D0 | Intra sign./D0 | Inter sign./D0 |
|---|---|---|---|---|---|---|
| Erythema | Treated | 3.35 | 3.23 | -0.12 | 0.257 | 0.02 |
| | Control | 3.57 | 3.57 | 0 | 1 | |
| Thickness | Treated | 3.46 | 2.96 | -0.50 | **0.002** | **0.011** |
| | Control | 3.38 | 3.33 | -0.05 | 0.317 | |
| Desquamation | Treated | 3.54 | 2.23 | -1.31 | **<0.001** | **<0.001** |
| | Control | 3.48 | 3.48 | 0 | 1 | |
| TSS score | Treated | 10.35 | 8.4 | -1.93 | **<0.001** | **<0.001** |
| | Control | 10.43 | 10.38 | -0.05 | 0.317 | |

Compared to the control area, the reduction observed in the thickness, the desquamation and the total TSS score was significantly greater than that observed on the control area.

Table 4 indicates the average results of the other clinical signs at D0 and D9 (after 5 applications). Figure 6 represents the change in these other clinical signs at D0 and D9 (after 5 applications).

**Table 4**

| | Area | Av. D0 | Av. D9 | Delta/D0 | Intra sign./D0 | Inter sign./D0 |
|---|---|---|---|---|---|---|
| Pruritus | Treated | 1.65 | 0.69 | -0.96 | **0.001** | **0.003** |
| | Control | 1.62 | 1.76 | 0.14 | 0.317 | |
| Hyperkeratosis | Treated | 0.88 | 0.38 | -0.50 | **0.017** | **0.017** |
| | Control | 0.95 | 0.95 | 0 | 1 | |
| Dryness | Treated | 3.85 | 3.50 | -0.35 | **0.024** | 0.336 |
| | Control | 3.86 | 3.76 | -0.10 | 0.317 | |
| Roughness | Treated | 3.89 | 2.85 | -1.04 | **<0.001** | **0.001** |
| | Control | 3.91 | 3.91 | 0 | 1 | |

At D9, i.e. after 5 applications, the pruritus, the hyperkeratosis, the dryness and the roughness were significantly improved and these changes were significantly different from the change observed on the control area for the pruritus, the hyperkeratosis and the roughness.

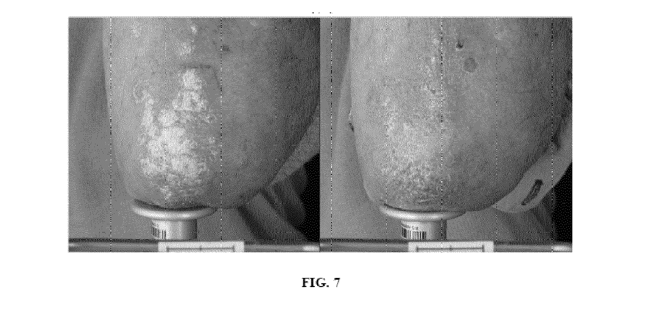

A reduction in the total TSS score was observed in 81% of cases. An improvement of the visual appearance of the plaque selected was observed for 85% of cases and an improvement of the pruritus was observed for 54% of cases. Figure 7 shows an example of the visual appearance of the skin before (D0) and after 5 applications (D9) of the product.

Comparison with other treatments: The volunteers were also requested to compare the gel with respect to treatments or other products that they were accustomed to using or that they had already used. Compared to Daivobet (topical application), the test product was judged to be equivalent or more effective in terms of itching, treatment time and scales, and equivalent in terms of redness. The gel of Example 1 was perceived by the majority as being more effective than the oral treatment Soriatane in terms of scales, itching, redness and treatment time.

The inventors have shown the effectiveness of an aqueous composition comprising at least 20% by weight of alkali metal bicarbonate in reducing the TSS score, in reducing the clinical signs of thickness, desquamation, pruritus, hyperkeratosis and roughness and in improving the visual appearance of the psoriasis plaques. The composition was also perceived to be effective by the volunteers in terms of the reduction in itching and the calming effect, much appreciated and well tolerated.

### Example 3: Formulation and effect of an antidandruff composition.

On the scalp of a person where the presence of dandruff is observed, 30 ml, i.e. around the equivalent of two tablespoons, of the topical gel produced in Example 1.b were applied over the whole of the scalp while gently massaging the skin with the gel at the roots of the hair. The composition was left to dry for a few minutes. After drying, the hair was rinsed with water so as to remove the composition according to the invention that had dried. The presence of soluble alkali metal bicarbonate enables easy dissolving and easy rinsing of the composition. At the end of the application and of the rinsing with water, an observation of the scalp made it possible to observe a great reduction or even an absence of dandruff after this operation.

### Example 4: Formulation of topical gels with organic gelling agent.

Example 4.a (conform): Formulation of a gel comprising: 30% of Bicar® Codex 0/13 pharmaceutical-grade sodium bicarbonate (Solvay), 5% glycerine (Cooper), 1.5% of gelling agent Solagum AX (Seppic) comprising Acacia Senegal Gum and Xanthan gum, 0.25% of gelling agent Napture (Sensient) comprising Acacia Senegal Gum, 0.5% conservative agents Sepecide HB (Seppic), and complemented to 100% with water (QSP).

After weighing the compounds, on a lab rotor-stator mixer at 1500 rpm and 20°C, glycerine was added to the water, then the conservative, then Bicar® was dispersed in the preceding mixture, and then gelling agents were introduced in about 10 minutes to progressively form the gel.

Analysis made: pH measurement, viscosity measurement on Physica® viscometer at 25°C (1 minute at 20 rpm), stability measurement: by centrifugation at 4000 rpm during 20 minutes.

The gel formed was white, fluid, with a viscosity of 1700 mPa.s, a pH 8.9, and stable after centrifugation.

Example 4.b (conform): Gel comprising: 30% of Bicar® Codex 0/13 pharmaceutical-grade sodium bicarbonate (Solvay), 5% glycerine (Cooper), 1.0% of gelling agent Solagum AX (Seppic) comprising Acacia Senegal Gum and Xanthan gum, 0.5% of gelling agent Napture (Sensient) comprising Acacia Senegal Gum, 0.5% conservative agents Sepecide HB (Seppic), and complemented to 100% with water (QSP).

Same manufacturing method was used to form the gel. The gel formed was white, fluid, with a viscosity of 2800 mPa.s, a pH 8.8, and stable after centrifugation.

Example 4.c (conform): A gel with same composition as example 4.b except that: sodium bicarbonate is replaced by potassium bicarbonate with similar particle size distribution to Bicar 0/13 and the gelling agent Napture is increased to 1% is produced with same manufacturing method. A white gel, fluid, is obtained.

### Example 5: Formulation of topical cream (conform).

A cream (emulsion) comprising 30% of Bicar® Codex 0/13 pharmaceutical-grade sodium bicarbonate (Solvay), 15% sweet almond oil (Olvea) as emolliant, 6% Emulium kappa (Gattefosse) comprising Candilla/jojoba/rice bran polyglyceryl-3 esters and glyceryl as emulsifying agent, 5% glycerine as humectant, 3 to 6 % Cetyl acohol (Cooper 04/14) as texturizing agent, 1% Protelan AGL-95 (Masso) comprising Sodium Lauroyl Glutamate stearate as anionic surfactant, 0.5% Solagum AX (Seppic) comprising Acacia and xanthan gum as gelling-thickener agent, 0.5% Sepecid HB (Seppic) as conservative agent.

After weighing the compounds, on a lab rotor-stator mixer at 1500 rpm and heated at 70°C, glycerine was added to the water, then Bicar® was dispersed in the preceding mixture, then gelling agents Solagum AX, and the anionic-surfactant were introduced progressively to form a first mixture at 70°C. A second mixture was prepared, mixing the emulsifying agent Emulium Kappa, sweet almond oil, and Cetyl acohol in a magnetic mixer heated also at 70°C. The second mixture was then dispersed in the first mixture on a lab rotor-stator mixer at 1500 rpm at 70°C. Then the composition was cooled at ambient air temperature, and the conservative was finally added in the composition.

The composition formed was a white cream, glossy, thick, presenting a uniform spread ability, which does not crack after application, with a viscosity of 6700 mPa.s, a pH 9.0, and the cream was stable after centrifugation test. The sodium bicarbonate particles could not be felt when the cream was spread on the skin.

## Claims

1. Method for the non-therapeutic cosmetic treatment of the skin, comprising the application to the skin of an aqueous composition comprising more than 20% by weight of alkali metal bicarbonate, advantageously at least 25%, preferably at least 29%, more preferably at least 30% by weight of alkali metal bicarbonate relative to the total weight of the composition, wherein the alkali metal bicarbonate is present in dissolved form and in particles form and the particles have a particle size distribution such that at least 50% by weight of the particles have a diameter of less than 100 µm.

2. Method according to Claim 1, in which the alkali metal bicarbonate is sodium bicarbonate.

3. Method according to either one of the preceding claims, in which the aqueous composition also comprises from 1 to 20% by weight of silica.

4. Method according to any one of the preceding claims, in which the aqueous composition is in the form of a gel, a cream or an ointment.

5. Method according to any one of the preceding claims, for improving the surface appearance of the skin.

6. Aqueous composition for cosmetic or pharmaceutical use, in particular dermatological use, to be applied on skin, comprising more than 20% by weight of alkali metal bicarbonate wherein the alkali metal bicarbonate is present in dissolved form and in particles form and the particles have a particle size distribution such that at least 50% by weight of the particles have a diameter of less than 100 µm for calming skin discomfort and/or for improving the surface appearance of the skin.

7. Aqueous composition according to Claim 6, for calming skin discomfort selected from itching, pruritus, tingling, tautness, and/or stinging.

8. Aqueous composition according to Claims 6 or 7, for a psoriatic skin, ichthyotic skin, hyperkeratotic skin and/or dry skin.

9. Aqueous composition according to any one of Claims 6 to 8 comprising, or consisting essentially of, or consisting of: 25 to 40% of alkali metal bicarbonate, 10 to 20% by weight of silica, and water.

10. Aqueous composition according to any one of Claims 6 to 8 comprising, or consisting essentially of, or consisting of:
- more than 20 and at most 55% of alkali metal bicarbonate,
- 1 to 5% of gelling agent such as polysaccharides, glycoproteins, xanthan gum, acacia gum, xylitylglucoside, arabinose, ribose,
- optionally 1 to 20% of silica,
- 1 to 8% of moisturizing agent such as glycerine, anhydroxylitol, xylitol,
- QSP water to 100% by weight.

11. Aqueous composition according to any one of Claims 6 to 10 in a gel form comprising less than 2% of oil, preferably essentially free of oil, and/or comprising less than 5% of organic acid or organic acid alkali metal salt, preferably essentially free of organic acid other than glycoproteins.

12. Aqueous composition according to any one of Claims 6 to 8 comprising, or consisting essentially of, or consisting of:
- more than 20 and at most 55% of alkali metal bicarbonate,
- 2 to 20% of oil such as almond oil,
- 3 to 10% of emulsifying agent such as Candilla/jojoba/rice bran polyglyceryl-3 esters and glyceryl stearate and cetearyl alcohol and sodium stearoyl lactylate,
- 0.1 to 2% of an anionic surfactant such as sodium lauroyl glutamate,
- optionally 1 to 20% of silica,
- 1 to 8% of moisturizing agent such as glycerine, anhydroxylitol, xylitol,
- 1 to 5% of gelling agent such as polysaccharides, glycoproteins, xanthan gum, acacia gum, xylitylglucoside, arabinose, ribose,
- QSP water to 100% by weight.

13. Aqueous composition according to any one of Claims 6 to 12 for treating psoriatic skin, ichthyotic skin, hyperkeratotic skin and/or dry skin.

14. Aqueous composition according to Claim 13 for being applied on a skin area to be treated and being left in contact with the skin area at least 10 minutes, advantageously at least 15 minutes, more advantageously at least 30 minutes and being then optionally rinsed.

15. Cosmetic non-therapeutic use of an aqueous composition according to any one of Claims 6 to 12, for improving the surface appearance of the skin.
